Europäisches Patentamt

**European Patent Office**

**Office européen des brevets**

(11) Numéro de publication: **0 014 150 B1**

(12) **FASCICULE DE BREVET EUROPEEN**

(45) Date de publication du fascicule du brevet:
25.01.84

(51) Int. Cl.³: **A 61 B 10/00**, A 61 B 17/22

(21) Numéro de dépôt: **80400105.5**

(22) Date de dépôt: **23.01.80**

---

(54) **Dispositif pour prélèvements d'endomètre.**

---

(30) Priorité: **24.01.79 FR 7901832**

(43) Date de publication de la demande:
**06.08.80 Bulletin 80/16**

(45) Mention de la délivrance du brevet:
**25.01.84 Bulletin 84/4**

(84) Etats contractants désignés:
**AT BE CH DE GB IT NL SE**

(56) Documents cités:
**US - A - 3 491 747**
**US - A - 3 635 222**

(73) Titulaire: **ARTS ET TECHNIQUES NOUVELLES Société à responsabilité limitée dite:, 156, rue Oberkampf, F-75011 Paris (FR)**

(72) Inventeur: **Augros, Jacques, Chemin de la Croix Baillet, F-95400 Villiers le Bel (FR)**

(74) Mandataire: **Boukhors, Alain et al, Cabinet BEAU de LOMENIE 55, Rue d'Amsterdam, F-75008 Paris (FR)**

---

Dispositif pour prélèvements d'endomètre

La présente invention concerne un dispositif pour prélèvements d'endomètre composé d'au moins un élément racleur allongé élastiquement déformable, de forme arquée à l'état libre, cet élément racleur étant fixé en bout d'une tige coulissant dans un tube à l'intérieur duquel ledit élément racleur peut être introduit par tirage de la tige dans le tube.

Dans ce dispositif on réintroduit l'élément racleur dans le tube, après usage, en tirant sur la tige afin de sortir le dispositif de la cavité raclée sans souiller le prélèvement.

Dans des dispositifs connus de ce type (brevets américains 3 635 222 et 3 491 747), l'élément racleur comporte des dents qui, lorsque ledit élément racleur est réintroduit dans le tube, raclent la paroi sur laquelle un endomètre doit être prélevé.

Dans ces dispositifs connus, les dents fléchissent durant l'opération de prélèvement et, de ce fait, elles raclent mal la paroi sur laquelle doit être fait le prélèvement, de sorte que l'on n'obtient pas de prélèvement important.

Le dispositif selon l'invention remédie à cet inconvénient et a notamment pour but de proposer un dispositif permettant un prélèvement plus important sans risque de blessure de la paroi raclée.

Ce but est atteint, conformément à l'invention, du fait que ledit élément racleur est constitué de plusieurs lamelles de raclage liées bout à bout entre elles par une partie étroite déformable de façon élastique, qu'il est incurvé, à l'état libre, dans le plan desdites lamelles, et que son cambrage est assuré par un jonc d'appui élastiquement déformable sous-tendant ledit élément racleur et cambré à l'opposé de ce dernier, moyennant quoi, lorsque ledit élément racleur muni du jonc est introduit dans une cavité d'un être biologique et que la tige est pivotée autour de son axe, l'élément racleur subit une torsion sensiblement héliocoïdale.

Avantageusement, la longueur du jonc est inférieure à celle de l'élément racleur.

Avantageusement, l'extrémité de l'élément racleur comporte une sphère de diamètre supérieur à celui du tube afin de former butée d'arrêt lors de la rentrée de cet élément racleur dans le tube.

Avantageusement, le bout de la tige opposé à l'élément racleur et qui est extérieur au tube, comporte au moins une languette qui forme butée d'arrêt pour le coulissement de la tige dans le tube afin d'être sûr qu'en butée, l'élément racleur est sorti du tube, cette languette s'aplatissant contre la tige lorsqu'on introduit celle-ci dans le tube.

Avantageusement, l'élément racleur, le jonc et la tige et, le cas échéant, la sphère et la languette de butée, forment ensemble une seule pièce moulée en matière plastique.

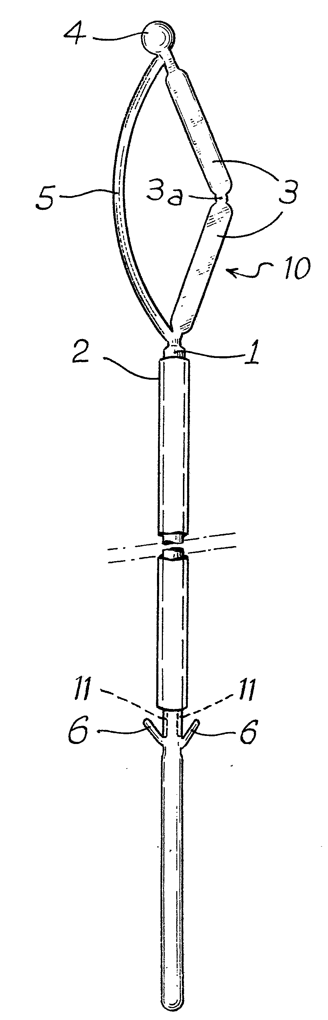

Les caractéristiques et avantages de l'invention seront mieux compris à la lecture de la description qui va suivre d'un exemple de réalisation et en se référant au dessin annexé sur lequel:

– la figure unique est une vue en élévation d'un dispositif selon l'invention.

Le dispositif de prélèvement selon l'invention est constitué par une tige 1 de longueur appropriée dont une des extrémités se termine par un dispositif racleur 10. Cette tige 1 coulisse dans un tube 2.

Le dispositif racleur 10 est constitué par deux lamelles 3 réunies par une partie étroite 3a déformable de façon élastique, formant charnière; les lamelles 3 forment un angle obtus entre elles, l'une des lamelles 3 est reliée à la tige 1 et l'autre à une sphère – ou boule – 4. Cette sphère 4 et la lame 3 du bas sont réunies par un jonc 5 en forme d'arc de cercle qui sous-tend l'arc formé par les lamelles 3; le jonc 5 tourne sa convexité du côté opposé aux lamelles 3; la longueur du jonc 5 est, de préférence, légèrement inférieure à celle des deux lamelles 3 mises bout à bout qui forment l'élément racleur proprement dit 10.

A l'autre extrémité de la tige 2, deux languettes 6 disposées de chaque côté, permettent le passage du tube 2 (pour le montage du dispositif) en se rabattant dans un logement 11 prévu à cet effet dans la tige 1. Ces languettes 6 forment, par la suite, butée limitant le coulissement de la tige 1 dans ce tube 2.

Pour l'utilisation du dispositif, l'on tient le tube 2 et l'on tire vers le bas de la tige 1, le dispositif de prise 3, 5, 10 pénètre dans le tube jusqu'à l'entrée en butée de la boule 4 contre le bout du tube 2, cette boule présentant un diamètre plus fort que celui du tube 2.

Il est ensuite aisé d'introduire le dispositif dans la cavité de l'être biologique sans recueillir au passage des éléments étrangers au prélèvement souhaité.

Lorsque le dispositif est en place, en repoussant la tige 1 dans le tube 2, le dispositif de prise 3, 5, 10 ressort et reprend sa forme initiale, les butées 6 empêchant un coulissement excessif de la tige 1 dans le tube 2. En tournant la tige 1 autour de son axe longitudinal dans le tube 2, un mouvement de rotation est imprimé au dispositif de prise et les lamelles 3, sous la pression due à l'appui de l'arc de cercle 5, peuvent ainsi racler et prélever la muqueuse à examiner. Cette combinaison d'appui et de rotation amène une torsion du dispositif de prise, obligeant les lamelles 3 à prendre une position hélicoïdale sous un certain angle procurant un prélèvement plus important, sans risque de blessure, causes de saignements qui rendent la lecture ultérieure du prélèvement difficile.

Après cette rotation, il suffit de rentrer le dispositif de prise dans le tube 2 en tirant la tige 1 jusqu'à l'entrée en butée de la boule 4, le prélèvement raclé se trouve alors protégé contre les contaminations inévitables, lors du retrait du lieu de prélèvement.

La manœuvre inverse est pratiquée ensuite et permet l'étalement du prélèvement sur une lame de microscope pour examen.

Le dispositif qui vient d'être décrit peut être fabriqué par moulage en matière plastique; les éléments 1, 3, 4, 5, 10 sont avantageusement moulés en une seule pièce. Ce dispositif permet, par sa conception, des prélèvements propres et précis sur une surface importante et sans traumatisme lors de ce travail.

Bien entendu diverses modifications peuvent être apportées par l'homme de l'art au dispositif qui vient d'être décrit uniquement à titre d'exemple, non limitatif, sans sortir du cadre de l'invention, en particulier on peut remplacer les deux lamelles 3 par une seule lamelle arquée soustendue par le jonc 5.

## Revendications

1. Dispositif pour prélèvements d'endomètre, composé d'au moins un élément racleur allongé (10) élastiquement déformable, de forme arquée à l'état libre, cet élément racleur étant fixé en bout d'une tige (1) coulissant dans un tube (2) à l'intérieur duquel ledit élément racleur peut être introduit par tirage de la tige dans le tube, caractérisé en ce que ledit élément racleur (10) est constitué de plusieurs lamelles de raclage (3) liées bout à bout entre elles par une partie étroite (3a) déformable de façon élastique, en ce qu'il est incurvé à l'état libre, dans le plan desdites lamelles, et en ce que son cambrage est assuré par un jonc d'appui élastiquement déformable (5) sous-tendant ledit élément racleur et cambré à l'opposé de ce dernier, moyennant quoi, lorsque ledit élément racleur (10) muni du jonc (5) est introduit dans une cavité d'un être biologique et que la tige (1) est pivotée autour de son axe, l'élément racleur (10) subit une torsion sensiblement hélicoïdale.

2. Dispositif selon la revendication 1, caractérisé en ce que la longueur du jonc (5) est inférieure à celle de l'élément racleur (10).

3. Dispositif selon l'une des revendications 1 ou 2, caractérisé en ce que l'extrémité libre de l'élément racleur (10) comporte une sphère (4) de diamètre supérieur à celui du tube (2) afin de former butée d'arrêt lors de la rentrée de cet élément racleur (10) dans le tube (2).

4. Dispositif selon l'une des revendications 1 à 3, caractérisé en ce que le bout de la tige (1) opposé à l'élément racleur (10) et qui est extérieur au tube (2) comporte au moins une languette (6) qui forme butée d'arrêt pour le coulissement de la tige (1) dans le tube (2) afin d'être sûr qu'en butée, l'élément racleur (10) est sorti du tube (2), cette languette (6) s'aplatissant contre la tige (1) lorsqu'on introduit celle-ci dans le tube (2).

5. Dispositif selon l'une des revendications 1 à 4, caractérisé en ce que l'élément racleur (10), le jonc (5) et la tige (1) et, le cas échéant, la sphère (4) et la languette de butée (6) forment ensemble une seule pièce moulée en matière plastique.

## Claims

1. Device for taking samples of endometrium, composed of a least one elongated scraper element (10), which is elastically deformable and bent when in the free state, said scraper element being fixed on the end of a rod (1) sliding in a tube (2) inside which the said scraper element may be introduced by pulling said rod in said tube, characterised in that said scraper element (10) is constituted of a plurality of scraping blades (3) connected together end to end by a narrow elastically deformable part (3a), in that it is incurved in the free state, inside the plane of said blades, and in that its arched shape is caused by an elastically deformable bow-shaped member (5) sub-tending the said scraper element and arched towards the opposite side thereof, so that when said scraper element (10) equipped with the bow-shaped member (5) is introduced into a cavity of the body and when the rod (1) is pivoted about its axis, the scraper element (10) is subjected to a substantially helical torsion.

2. Device according to claim 1, characterised in that the length of the bow-shaped member (5) is less than that of the scraper element (10).

3. Device according to any one of claims 1 or 2, characterised in that the free end of the scraper element (10) comprises a sphere (4) of diameter greater than that of the tube (2) so as to form a stop when said scraper element is returned inside the tube (2).

4. Device according to any one of claims 1 to 3, characterised in that the end of the rod (1) opposite the scraper element (10) and which is outside the tube (2) comprises at least a tongue (6) which forms a stop for the sliding of the rod (1) inside the tube (2) in order to ensure that, in the stop position, the scraper element (10) is out of the tube (2), said tongue (6) flattening against the rod (1) when the latter is introduced into said tube (2).

5. Device according to any one of claims 1 to 4, characterised in that the scraper element (10), the bow-shaped member (5) and the rod (1) and, if necessary, the sphere (4) and the stop tongue (6) for together a single piece molded from plastic material.

## Patentansprüche

1. Vorrichtung für Endometriumabstriche, bestehend aus zumindest einem elastisch verformbaren, länglichen Abstreifelement (10) von im freien Zustand bogenförmiger Gestalt, welches Abstreifelement am Ende eine Stange (1) befestigt ist, die in einem Rohr (2) gleitet, in dessen Inneres das Abstreifelement durch Einziehen der Stange in das Rohr einführbar ist, dadurch gekennzeichnet, dass das Abstreifelement (10) aus mehreren, an ihren Enden durch einen geraden, elastisch verformbaren Abschnitt (3a) miteinander verbundenen Abstreiflamellen (3) gebildet ist, dass es im freien Zustand in der Ebene der Lamellen gekrümmt ist und dass seine Krümmung durch einen elastisch verformbaren Haltestab (5) gewährleistet ist, der das Abstreifelement unter Spannung hält und gegenüber letzterem gekrümmt ist, mit der Massgabe, dass bei Einführen des mit dem Stab (5) versehenen Abstreifelements (10) in eine Körperhöhle eines Lebewesens und bei Drehen der Stange (1) um ihre Achse das Abstreifelement (10) eine im wesentlichen schraubenförmige Verdrehung erfährt.

2. Vorrichtung nach Anspruch 1, dadurch gekennzeichnet, dass die Länge des Stabes (5) geringer als jene des Abstreifelements (10) ist.

3. Vorrichtung nach einem der Ansprüche 1 oder 2, dadurch gekennzeichnet, dass das freie Ende des Abstreifelements (10) zur Bildung eines Halteanschlags beim Einziehen dieses Abstreifelements (10) in das Rohr (2) eine Kugel (4) von einem grösseren Durchmesser als jener des Rohres (2) umfasst.

4. Vorrichtung nach einem der Ansprüche 1 bis 3, dadurch gekennzeichnet, dass das dem Abstreifelement (10) gegenüberliegende und ausserhalb des Rohres (2) befindliche Ende der Stange (1) zumindest eine Zunge (6) umfasst, die einen Halteanschlag für die Gleitbewegung der Stange (1) im Rohr (2) bildet, um sicher zu sein, dass im Anschlag das Abstreifelement (10) ausserhalb des Rohres (2) ist, wobei sich diese Zunge (6) an die Stange (1) anschmiegt, wenn diese in das Rohr (2) eingeführt wird.

5. Vorrichtung nach einem der Ansprüche 1 bis 4, dadurch gekennzeichnet, dass das Abstreifelement (10), der Stab (5) und die Stange (1), und gegebenenfalls die Kugel (4) und die Anschlagzunge (6), zusammen ein einteiliges, aus Plastikmaterial gegossenes Stück bilden.

5